# EUROPEAN PATENT SPECIFICATION

(11) **EP 0 357 027 B1**
(45) Date of publication and mention of the grant of the patent: **10.05.1995**
(21) Application number: 89115996.4
(22) Date of filing: 30.08.1989
(51) Int. Cl.: C12Q 1/26, C12M 1/40, C12N 9/96

(54) **Alcohol oxidase enzyme electrode and its use for quantitative alcohol determination.**
Alkohol-oxidase Enzymelektrode und Verfahren zur Bestimmung von Alkoholeinhalt
Electrode à alcohol oxidase et procédé pour déterminer la teneur alcoolique utilisant cell-ci.

(30) Priority: 30.08.1988 JP 217641/88; 31.08.1988 JP 218844/88
(43) Date of publication of application: 07.03.1990
(73) Proprietor: NEW OJI PAPER CO., LTD., Tokyo (JP)
(72) Inventor: Hayashi, Ryuzo, Higashiosaka-shi Osaka 577 (JP); Kariyone, Akio, Shimogyo-ku, Kyoto-shi, Kyoto 600 (JP); Hashizume, Yoshio, Kakogawa-shi Hyogo-ken 675 (JP)
(74) Representative: Beetz & Partner Patentanwälte

(56) References cited:
- EP-A- 0 133 481
- EP-A- 0 164 008
- DD-A- 239 045
- US-A- 4 430 427
- US-A- 4 556 635
- P.W. CARR et al., "Immobilized Enzymes in Analytical & Clinical Chemistry", 1980, John Wiley & Sons, New York, NY (US); p. 166#
- BIOSIS, vol. 74, 1982; T.H. CROMARTY, no. 74049504#
- ANALYTICAL CHEMISTRY, vol. 55, no. 9, August 1983, Columbus, OH (US); G.G. GUILBAULT et al., pp. 1582-1585#

## Description

The present invention relates to a highly sensitive and stable alcohol oxidase enzyme electrode and its use in a method for the quantitative determination of the alcohol content with high accuracy and high sensitivity.

Measuring instruments employing immobilized enzyme working electrodes are known for handiness, rapidity, substrate specificity and other features, and are widely used for many applications, including clinical analysis, food analysis and environmental measurements. Among others, the amperometric measurement, that is, the detection of an increase or decrease of the electrode active substance caused by the enzyme reaction as a change in the current output from the working electrode to which a constant voltage is applied, allows to achieve high sensitivity and excellent stability. Accordingly, various electrodes, instruments and methods for the amperometric measurement have been developed.

As representative amperometric methods, the measuring by means of an oxygen electrode and the measuring by means of a hydrogen peroxide electrode are known. These two methods are most widely employed with use of immobilized enzyme electrodes, the hydrogen peroxides electrode being superior to the oxygen electrode in response speed and S/N ratio.

In the fields of food, fermentation and clinical analysis, there are keen demands for the development of methods and apparatus for the determination of alcohol, especially ethanol, and various proposals have been disclosed so far. For example, US 4 556 635 discloses the use of an enzyme electrode comprising an immobilized alcohol oxidase for the determination of the alcohol content in water insoluble solvents. However, the activity of the alcohol oxidase which is an enzyme catalyzing the oxidation of ethanol is relatively low, and its stability is not sufficient in practice.

In the initial research phase, alcohol oxidase was used without immobilizing because the activity of alcohol oxidase is low, and a method of detecting the catalytic reaction of the enzyme in the solution by means of an enzyme electrode was proposed (G.G. Guilbault, G.J. Lubrano: Anal. Chim. Acta, 69, 189, 1974). In this report it is stated that hydrogen peroxide is hard to detect with the hydrogen peroxide electrode which is produced by the alcohol oxidase. In this regard, however, no details have been reported yet.

Afterwards, immobilized enzyme electrodes obtained by immobilizing alcohol oxidase have been reported. This prior art includes a method of immobilizing alcohol oxidase by a mild reaction with a crosslinking agent such as glutaraldehyde while preventing the inactivation of the alcohol oxidase as far as possible by the presence of a polymer more likely to react with the crosslinking agent than with the alcohol oxidase, and a method using a polymer which is made insoluble by pH changes (JP-A-60-176587 and 62-215387). These methods are effective to a certain degree to prevent the inactivation of the alcohol oxidase, but are not satisfactory regarding the bonding force of the alcohol oxidase, which is not sufficiently immobilized, thus involving the possibility of an enzyme leakage. Besides, these methods are not suited for solving the fundamental problems of the low activity of the alcohol oxidase, in particular, the low activity for producing hydrogen peroxide, as mentioned above, and the insufficient stability of immobilized alcohol oxidase.

Further, these methods could not improve the thermal stability of enzyme electrodes obtained by immobilizing alcohol oxidase. The thermal stability is often used as an index for the stability of an enzyme, and enzymes to be used in enzyme electrodes should preferably possess the highest possible thermal stability. Of course, if the enzyme is thermally unstable, it is possible to operate the enzyme electrode at low temperature, however, at low temperature the enzyme activity is lowered, and it is likely that the sensitivity is lowered. Moreover, the temperature control of the instrument near room temperature is relatively difficult because of fluctuations of the room temperature. Therefore it is desired to dispose of enzyme electrodes which may be operated stably at temperatures about 10 °C or more higher than room temperature. Alcohol oxidase, however, is generally inferior in its thermal stability, and it is difficult to achieve a stable operation of alcohol oxidase enzyme electrodes at such temperatures.

It is hence the object of the invention to provide an enzyme electrode comprising stably and firmly immobilized alcohol oxidase and a sensitive method for the quantitative determination of alcohol by using this enzyme electrode and detecting oxygen or hydrogen peroxide in stable measurements. Furthermore, the thermal stability of the immobilized alcohol oxidase enzyme electrode shall be improved.

The above object is achieved according to the independent claims. The dependent claims relate to preferred embodiments.

The alcohol oxidase enzyme electrode of the invention comprises an immobilized enzyme layer on a membrane or on a conductive electrode surface and is obtainable by applying a solution containing an alcohol oxidase, a crosslinking agent and reduced glutathione to the membrane or the conductive electrode surface.

Reduced glutathione has been known as a stabilizer for alcohol oxidases which reacts with peroxides, as mentioned in EP-A-133 481. For this purpose, reduced glutathione is used in large amounts which interfere with the quantitative determination of hydrogen peroxide produced by that enzyme.

According to EP-A-133 481, which deals with disposable colorimetric ethanol test strips comprising alcohol oxidase and an oxygen acceptor giving a colored reaction product, reduced glutathione may also be used for preventing the undesired autoxidation which yields hydrogen peroxide, leading to false endpoints, wherein the reduced glutathione is added in a "controlled neutralizing amount" for neutralizing the peroxide functionality arising from autoxidation.

In contrast to this prior art, reduced glutathione is used according to the present invention during the immobilization of the alcohol oxidase with the crosslinking agent.

The alcohol oxidase is an enzyme produced by basidiomycetes, yeast, etc., and it is known to exist particularly abundantly in the peroxisomes of methanol-utilizing microorganisms. The present inventors investigated the characteristics of alcohol oxidase, and made various attempts to develop methods of immobilization and measurement.

In the first place, in order to prepare an immobilized enzyme membrane as rigid as possible, aldehydes were used as the crosslinking agent for covalently binding the alcohol oxidase. Alcohol oxidase is a comparatively large enzyme with a molecular weight exceeding 300,000 and it has been found that the bonding force is weak in immobilization by covalent bonds. The reason is presumed by that only a few amino groups of the alcohol oxidase are exposed on the molecule surface, although alcohol oxidase has a comparatively large molecular weight, or that there are only a small number of amino acids having amino groups capable of bond formation with the crosslinking agent, for example glutaraldehyde.

Therefore, in order to immobilize alcohol oxidase rigidly, it is necessary to raise the concentration of the crosslinking agent. However, when such a measure is taken, the enzyme activity drops suddenly, that is, the enzyme is inactivated.

To prevent such an inactivation, the stability has been investigated by using various additives such as dithiothreitol, cysteine, and reduced glutathione. As a result, glutathione was found to be suited for this purpose, and it was also found that an immobilized enzyme membrane excellent in strength was obtained. The reason for the prevention of inactivation of the alcohol oxidase by the use of reduced glutathione is not clearly known, but the following mechanism is known. Intrinsically, alcohol oxidase is inactivated by copper or mercury ions, and it is presumed that the thiol group in the alcohol oxidase molecule takes part in the enzyme reaction. Therefore, during the crosslinking reaction by aldehydes it is highly possible that the thiol group which is in the active site of the molecule, reacts with aldehyde and thus becomes inactivated. However, by the presence of reduced glutathione which is an oligopeptide possessing a thiol residue, it is considered that the thiol group in the molecule of the alcohol oxidase is protected so that the inactivation is prevented. The present invention is based on this finding.

The quantity of addition of reduced glutathione in the alcohol oxidase solution is preferably within the range of 0.1 to 10 mM. If the concentration is too low, sufficient effect is not to be expected, and if it is too high, the immobilization is impeded. The reason therefor seems to be that the free amino group in the molecule of reduced glutathione reacts with aldehyde, thus lowering the effective aldehyde concentration.

As the alcohol oxidase, the enzymes derived from methanol-utilizing microorganisms (methanol-utilizing yeast, methanol-utilizing bacteria, etc.), especially methanol-utilizing Candida yeast and methanol-utilizing Pichia yeast, are preferable under the aspect of activity retention. Alcohol oxidase is contained abundantly in the peroxisomes (microbodies) of methanol-utilizing micro-organisms.

For the immobilization of alcohol oxidase using aldehyde, it is desired to heat above room temperature in order to raise the reaction rate and to form a rigid crosslinking film. However, the enzymes derived from basidiomycetes may be inactivated when heated over 25 °C. In contrast thereto, the enzymes derived from methanol-utilizing microorganisms relatively excel in heat resistance, and can withstand about 40 °C if only for a short time.

In particular, from the viewpoint of heat resistance, the enzymes derived from methanol-utilizing Candida yeast and methanol-utilizing Pichia yeast are excellent.

As the crosslinking agent, aldehydes are used, preferably multifunctional aldehydes, for example, formaldehyde, glyoxal, and glutaraldehyde; glutaraldehyde is particularly preferably used because the activity lowering is less.

The concentration of aldehyde in the alcohol oxidase solution is preferably 0.1 to 1.0 wt.%, and more preferably 0.2 to 0.7 wt.%. If the aldehyde concentration is too low, the strength of the immobilized enzyme membrane is lowered, and if it is too high, lowering of the activity cannot be prevented sufficiently even if the reduced glutathione is added.

Furthermore, by coexistence of at least one type of protein other than alcohol oxidase, at the time of reaction with the crosslinking agent, the aforesaid proteins penetrate into gaps of the large alcohol oxidase molecules, thereby forming more rigid membranes of immobilized enzyme. To achieve this object, it is desired to use proteins of relatively low molecule weight and high amino group content. For example, gelatine, albumin, globulin, polylysine, and polyarginine conform to these conditions, and in particular gelatine and albumin are preferable under the aspect of price. These proteins get into gaps of the large alcohol oxidase molecules and are bonded with several multifunctional aldehyde molecules of the crosslinking agent on the molecule surface of the proteins, which is considered to cause an increased number of crosslinking points of the multifunctional aldehyde with the alcohol oxidase. The weight of the protein to be added at the time of immobilization is in practice preferably 1/10 to about 10 times the weight of alcohol oxidase. If this content is too small, there is no effect, and if too much, the alcohol oxidase is diluted, and the sensitivity is lowered.

The alcohol oxidase may be directly immobilized on the electrode, which may be made of platinum or of another conductive material, or may be immobilized on a permselective membrane, e.g. a membrane having selective permeability for hydrogen peroxide for removing disturbing substances such as ascorbic acid.

As the permselective membranes, various kinds of membranes, for example, crosslinked membranes of albumin and glutaraldehyde, acetylcellulose membranes, and the like, may be used.

The enzyme electrode prepared in this way has not only high activity when used for detecting the consumption of oxygen (oxygen electrode) but also high activity when used for detecting hydrogen peroxide (hydrogen peroxide electrode), as will be described below.

That is to say, in the previous method, the activity may not be always sufficient when detecting hydrogen peroxide. For determining the reason therefor, hydrogen peroxide was measured instead of alcohol, and it was found that the sensitivity of the electrode for hydrogen peroxide was low. Furthermore, in connection with the investigation of the activity of the alcohol oxidase electrode to decompose hydrogen peroxide, an obvious hydrogen peroxide decomposing activity, that is, a catalase activity, was recognized. This is because a hard-to-refine catalase is contained in the alcohol oxidase. Therefore, in order to obtain an excellent hydrogen peroxide electrode, it is preferable to suppress only the catalase activity. To specifically suppress the catalase activity, it is effective to use cyanide or azide ions, and the like, however, the cyanide ion is not desired owing to its toxicity.

Accordingly, sodium aside was added to the buffer solution, in which the enzyme reaction was performed in solution. As a result, the catalase activity was evidently lowered, but the alcohol oxidase activity was also lowered. A disturbance of the activities of catalase and alcohol oxidase could be observed when about 0.01 mM of sodium azide was added to the reaction system, and the alcohol oxidase activity was reduced by about 5 to 10 % when about 0.1 mM was added.

On the other hand, the enzyme electrode comprising covalently bond immobilized alcohol oxidase has a remarkably improved resistance to aside ions, and the alcohol oxidase activity is hardly suppressed while it was found that the catalase activity is effectively inhibited.

When sodium azide is added to the buffer solution used in the measurement using the enzyme electrode, for example, a 100 mM sodium phosphate buffer solution of pH 7.5 in a concentration of 0.1 to 1 mM, only the catalase activity can be suppressed. If the sodium azide concentration is too low, the catalase activity cannot be suppressed sufficiently, or if it is too high, the alcohol oxidase activity is also impeded.

Meanwhile, when an immobilized enzyme electrode or an immobilized enzyme membrane is dipped into a buffer solution containing azide ions in a higher concentration, for example, a buffer solution containing 100 mM of azide sodium, and is left standing overnight at about 4 °C, and is then returned into a buffer solution free of sodium azide and left standing for about 5 h at room temperature, the catalase activity is lost, and the alcohol oxidase activity is recovered to about 70 %. This is considered to be due because the resistance of catalase to azide is not changed so much although the resistance of the alcohol oxidase to azide is increased by the immobilization. However, since this method is too time-consuming it is desisred to always add sodium azide at low concentration to the measuring system in practice.

In accordance with still a further embodiment, a buffer solution containing polyacrylic acid is used for the measurement.

Preferably, the concentration of the polyacrylic acid to be added is within the range of 0.001 to 0.5 wt.%.

The electrochemical method for the present invention for the quantitative determination of the alcohol content of a sample in a buffer solution is based on contacting an enzyme electrode comprising immobilized alcohol oxidase with the solution and measuring the current due to oxygen consumed or due to hydrogen peroxide produced at the time of alcohol oxidation; it is characterized in that an enzyme electrode as defined above is used.

In addition, the following experiment was conducted in order to enhance the heat resistance of the enzyme electrode comprising immobilized alcohol oxidase. For example, the use of alcohol oxidase of Candida boidinii, which is a methanol-utilizing yeast, will be explained. In order to check the heat resistance of this enzyme, in the first place, the reaction of the solution system was conducted. The enzyme was dissolved in 100 mM sodium phosphate buffer solution (pH 7.5), and a specific amount of ethanol, and a solution containing horseradish peroxidase (hereinafter referred to as POD), phenol and 4-aminoantipyrine were added, and the reaction was conducted at 30 °C for a specific time. In this system, ethanol was oxidized into acetaldehyde by the reaction of the alcohol oxidase, and hydrogen peroxide was produced at the same time. The produced hydrogen peroxide was immediately decomposed by POD, and a red pigment is produced at this time by phenol and 4-aminoantipyrine. The amount of this red pigment was proportional to the produced quantity of hydrogen peroxide, i.e., proportional to the oxidized amount of ethanol; by measuring the absorbance of the reaction solution around 500 nm, the activity of the alcohol oxidase could be determined. In this way, the initial enzyme activity was measured. Thereafter, only the enzyme solutions were held at 35 °C, 37 °C, 39 °C, 41 °C, 43 °C, and 45 °C, respectively, for 30 min, and the enzyme activity of each solution was measured again at 30 °C. As a result, there was no significant change in the activity up to 37 °C, but at 39 °C, the activity was lowered to 70 % and at 41 °C to 30 % of the initial activity and at 45 °C, the activity was lost almost completely.

Next, alcohol oxidase and bovine serum albumine were mixed in a weight ratio of 1:1, and dissolved in a buffer solution, and glutaraldehyde was added in an amount of 0.5 % by weight; the mixed solution was applied onto a polished platinum electrode, and was allowed to become dry and solidified. Using this electrode as the working electrode, a platinum plate of 1 cm² was used as counter electrode, and a saturated calomel electrode (hereinafter referred to as SCE) was used as reference electrode, and they were dipped in a 100 mM sodium phosphate buffer solution controlled at a temperature of 30 °C, and a voltage of 0.6 V against the SCE was applied to the working electrode by using a potentiostat. To this system, a specific amount of ethanol was added, and the oxidation current of the hydrogen peroxide formation was recorded. By exchanging the buffer solution, the temperature of the system was elevated in the same steps as the solution system, and 30 min after the system had reached the respective temperatures, similar recordings were conducted. In this case, along with the temperature rise, not only the enzyme activity but also the sensitivity of the electrode to hydrogen peroxide varies, and therefore the operation temperature limit is higher than for the enzyme in solution explained above; at 41 °C, the activity was similarly lowered to 50 % of the initial sensitivity.

An enzyme electrode prepared with addition of 0.1 wt.% of polyacrylic acid to the buffer solution was repeatedly measured, and the initial sensitivity could be maintained up to 43 °C, and at 45 °C, 90 % of the initial sensitivity was retained.

In contrast thereto, the measurement of the solution system, using a buffer solution with addition of 0.1 wt.% of polyacrylic acid has shown that the heat resistance was not improved.

Thus, in the case of immobilized alcohol oxidase, by adding polyacrylic acid to the buffer solution used in the measurement, the stability of the enzyme can be enhanced.

This effect obtained by addition of polyacrylic acid according to this invention as described above is a novel phenomenon observed only in immobilized enzyme systems of the present invention, i.e. with immobilized enzyme electrodes prepared by applying an alcohol oxidase solution containing reduced glutathione. The polyacrylic acid to be added to the buffer solution preferably has a mean molecular weight of about 10 000 to 1 000 000, and the concentration preferably lies within the range of 0.001 to 0.5 wt.%. If the concentration is less than 0.001 wt.%, no sufficient effect occurs; if it is too high, the sensitivity may be lowered. The sensitivity drop at high concentrations of polyacrylic acid is considered to be due to the impediment of diffusion of the substrate because of elevated solution viscosity, or lowering of the activity of the enzyme itself.

The reason for the stabilizing effect of polyacrylic acid on an immobilized alcohol oxidase system is not clearly known. Anyway, it is presumed that certain changes occur in the conformation of the alcohol oxidase by the immobilization which favour the interaction with polyacrylic acid, thereby becoming thermally stable in the form of an alcohol oxidase-polyacrylic acid complex.

In the following, the invention will be explained with more details with reference to the drawings.
- Fig. 1: shows a flow type measuring apparatus used in individual embodiments;
- Fig. 2: shows measurement results of Example 6 (○ mark) and of References Example 2 ( 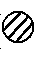 mark);
- Fig. 3: shows measurement results of Example 7 (○ mark) and of Reference Example 3 ( 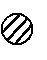 mark); and
- Fig. 4: shows measurement results of Example 8 (○ mark) and of Reference Example 4 ( 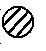 mark).

Referring now to the drawings, some of the preferred ambodiments of the invention are described in details below. The invention, however, is not limited to these embodiments. In the following description, % denotes wt.%.

### Example 1

### (1) Electrode preparation

The side surface of a platinum wire of 2 mm in diameter was coated with a heat-shrinkable PTFE, and one end of the wire was smoothly finished by using a file and an emery paper of 1500 count. Using this platinum wire as working electrode, a 1 cm² platinum plate as counter electrode and a saturated calomel electrode (SCE) as reference electrode, an electrolysis was conducted for 10 minutes in 0.1 M sulfuric acid at +1.4 V. Then the platinum wire was washed well in water, dried for 10 minutes at 40 °C, and was dipped for 1 hour into an anhydrous toluene solution containing 10 % of γ-aminopropyl triethoxy silane, and washed.

An enzyme was immobilized on this amino-silane coated platinum wire in the following manner.

5 mg of alcohol oxidase (Sigma Co.; derived from Candida boidinii) and 5 mg of bovine serum albumin (Sigma Co.; Fraction V) were dissolved in 1 ml of 100 mM sodium phosphate buffer solution (pH 7.5), and 1 mM of reduced glutathione and 0.5 % of glutaraldehyde were added. This mixed solution was promptly put on the prepared platinum wire, and was dried and cured at 40 °C for 30 minutes. Afterwards, the product was stored in 100 mM sodium phosphate buffer solution (pH 7.5).

### (2) Measuring method

Using the prepared enzyme electrode as working electrode, a 1 cm² platinum plate electrode as counter electrode, and an SCE as reference electrode, connected to a potentiostat, a voltage of 0.6 V against the SCE was applied to the working electrode. The buffer solution used in the measurement was 100 mM sodium phosphate buffer solution (pH 7.5) containing 50 mM of potassium chloride. The measuring temperature was 30 °C (± 0.2 °C). While stirring the solution with a magnetic stirrer, ethanol (1 mM) was added to the system, and the increase of the current output was recorded with a recorder.

Plotting a calibration curve for 1 to 10 mM of ethanol, the sensitivity of the electrode per 1 mM of ethanol was determined from the slope of the calibration curve. The sensitivity of 20 separately prepared electrodes was measured, and the mean and standard deviation values were calculated (Table 1). It was thus found that the electrodes obtained by the above-described method are stable and have a high sensitivity.

### Reference Example 1

### (1) Electrode preparation

The electrodes were prepared in the same manner as in Example 1, except that the reduced glutathione was not used when producing the electrodes.

### (2) Measuring method

The measurements were carried out in the same manner as in Example 1.

From the slope of the calibration curve, the sensitivity of the electrode per 1 mM of ethanol was determined. The sensitivity of 20 separately prepared electrodes and the mean and standard deviations are shown in Table 1 in comparison with the results of Example 1. It becomes evident that the electrodes prepared in this manner have a sensitivity of only one-third of that of Example 1. Thus, the effect of addition of reduced glutathione is confirmed.

**Table 1**

| Measuring example | Electrode sensitivity/mean ± standard deviation (»A/mM ethanol) |
|---|---|
| Example 1 | 0.210 ± 0.011 |
| Reference Example 1 | 0.072 ± 0.008 |

### Example 2

### (1) Electrode preparation

Electrodes were prepared in the same manner as in Example 1.

### (2) Measuring method

Using the prepared enzyme electrode as working electrode, a 1 cm² platinum plate electrode as counter electrode, and an SCE as reference electrode, connected to a potentiostat, a voltage of 0.6 V against the SCE was applied to the working electrode. The buffer solution used in the measurement was 100 mM sodium phosphate buffer solution (pH 7.5) containing 50 mM of potassium chloride. The measuring temperature was 30 °C (± 0.2 °C). While stirring the solution with a magnetic stirrer, ethanol (1 mM) was added to the system; and the increase of the current output was recorded with a recorder.

From the slope of the calibration curve, the sensitivity of the electrode per 1 mM of ethanol was determined. Next, the buffer solution for measurement was exchanged with a fresh one, and the solution was continuously stirred for 5 hours, and the sensitivity was measured again. The results are shown in Table 2. As becomes clear from Table 2, the sensitivity of the electrode is not changed by such a method of preparation.

### Example 3

### (1) Electrode preparation

Electrodes were prepared in the same manner as in Example 1, except that the bovine serum albumin was not used, and 10 mg of alcohol oxidase were used instead of 5 mg.

### (2) Measuring method

The measurements were carried out in the same manner as in Example 2.

From the slope of the calibration curve, the sensitivity of the electrode per 1 mM of ethanol was determined. The buffer solution for measurement was exchanged with a fresh one, and the solution was continuously stirred for 5 hours, and the sensitivity was measured again. The results are shown in Table 2. As becomes evident from Table 2, the sensitivity of the electrode prepared in this manner was lowered. By visual observation, separation of the immobilized enzyme membrane was recognized, and it is seen that the strength of the immobilized membrane is slightly inferior if no protein other than alcohol oxidase, such as albumin, is present.

**Table 2**

| Measuring example | Electrode sensitivity (»A/mM ethanol) | |
|---|---|---|
| | Upon start of measurement | After 5 h stirring |
| Example 2 | 0.205 | 0.203 |
| Example 3 | 0.151 | 0.020 |

### Example 4

### (1) Electrode preparation

Electrodes were prepared in the same manner as in Example 1.

### (2) Measuring method

Using the prepared enzyme electrode as working electrode, a 1 cm² platinum plate electrode as counter electrode, and an SCE as reference electrode, connected to a potentiostat, a voltage of 0.6 V against the SCE was applied to the working electrode. The buffer solution used in the measurement was a 100 mM sodium phosphate buffer solution (pH 7.5) containing 50 mM of potassium chloride with 10 »M of sodium azide. The measuring temperature was 30 °C (± 0.2 °C). While stirring the solution with a magnetic stirrer, ethanol (1 mM) was added to the system, and the increase of the current output was recorded with a recorder. Similarly, hydrogen peroxide with a known concentration was measured. From the slope of the calibration curve, the sensitivity of the electrode per 1 mM of ethanol or 1 mM of hydrogen peroxide was determined. The sensitivity of 20 separately prepared electrodes was measured, and the mean and standard deviation values were calculated (Table 3). With this measuring method, the sensitivity obtained concerning ethanol was about 5 times as high as in Example 1. Furthermore, as compared with the results of Example 5 mentioned below, the sensitivity to hydrogen peroxide is also enhanced. Thus, the effect of addition of sodium azide has been confirmed.

### Example 5

### (1) Electrode preparation

Electrodes were prepared in the same manner as in Example 1.

### (2) Measuring method

The measurements were carried out in the same manner as in Example 4, except that sodium azide was not added to the buffer solution.

From the slope of the calibration curve, the sensitivity of the electrode per 1 mM of ethanol or 1 mM of hydrogen peroxide was determined. The results are shown in Table 3. Evidently, both for ethanol and hydrogen peroxide, the sensitivity is low as compared with Example 4. This is because hydrogen peroxide is decomposed by catalase.

**Table 3**

| Measuring example | Electrode sensitivity/mean±standard deviation (»A/mM) | |
|---|---|---|
| | Ethanol | Hydrogen peroxide |
| Example 4 | 1.022 ± 0.050 | 6.150 ± 0.180 |
| Example 5 | 0.207 ± 0.010 | 1.170 ± 0.071 |

### Example 6

### (1) Electrode preparation

The side surface of a platinum wire of 2 mm in diameter was coated with a heat-shrinkable PTFE, and one end of the wire was smoothly finished by using a file and an emery paper of 1500 count. Using this platinum wire as working electrode, a 1 cm² platinum plate as counter electrode, and a saturated calomel electrode (SEC) as reference electrode, an electrolysis was conducted for 10 minutes at +1.4 V in 0.1 M sulfuric acid. Then the platinum wire was washed well in water, dried for 10 minutes at 40 °C, and was dipped in for 1 hour to an anhydrous toluene solution containing 10 % of γ-aminopropyl triethoxy silane, and washed. On this amino-silane coated platinum wire, an enzyme was immobilized in the following manner.

5 mg of alcohol oxidase (Sigma Co.; derived from Candida boidinii) and 5 mg of bovine serum albumin (Sigma Co.; Fraction V) were dissolved in 1 ml of 100 mM sodium phosphate buffer solution (pH 7.5), and 0.5 % of glutaraldehyde was added. 5 »l of this mixed solution was promptly put on the prepared platinum wire, followed by drying and curing for 30 minutes at 40 °C. Then it was stored in 100 mM sodium phosphate buffer solution (pH 7.5).

### (2) Measuring method

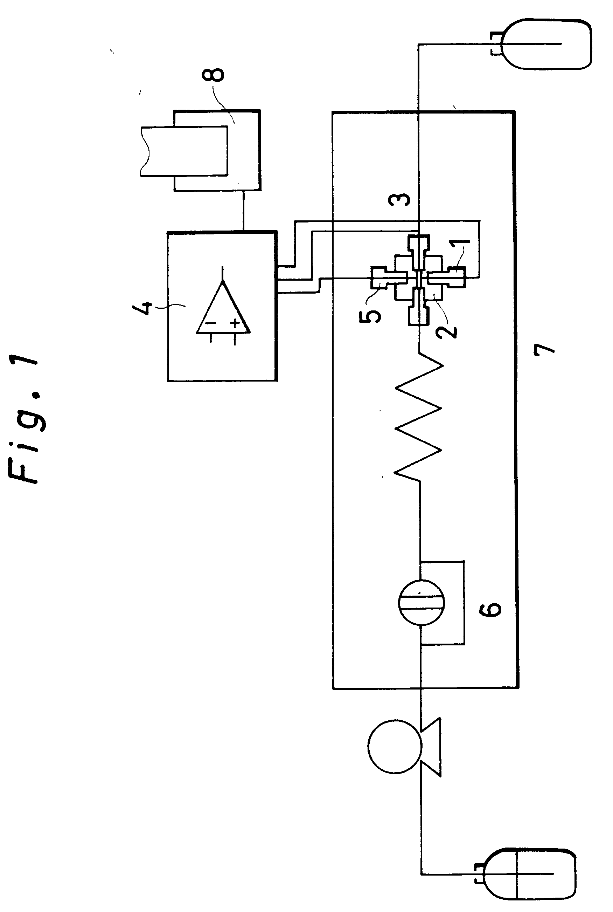

The prepared enzyme electrode was used as working electrode and incorporated into a flow type measuring apparatus shown in Fig. 1. An Ag|AgCl reference electrode 1 was used as reference electrode, and a stainless steel pipe connected to the outlet of the flow cell 2 was used as counter electrode 3. These three electrodes were connected to a potentiostat 4. To the working electrode 5, a voltage of 0.6 V against the Ag|AgCl reference electrode was applied. The line after the injection port 6 of the flow-type measuring apparatus was put into a thermostat 7. The buffer solution used in the measurement was 100 mM sodium phosphate buffer solution (pH 7.5) containing 50 mM of potassium chloride and 0.01 % of polyacrylic acid.

Initially, the measuring temperature was 30 °C (± 0.2 °C). From the injection port 6, 5 »l of a 50 mM aqueous ethanol solution were introduced, and the current output was recorded in a recorder 8, and the peak current was read.

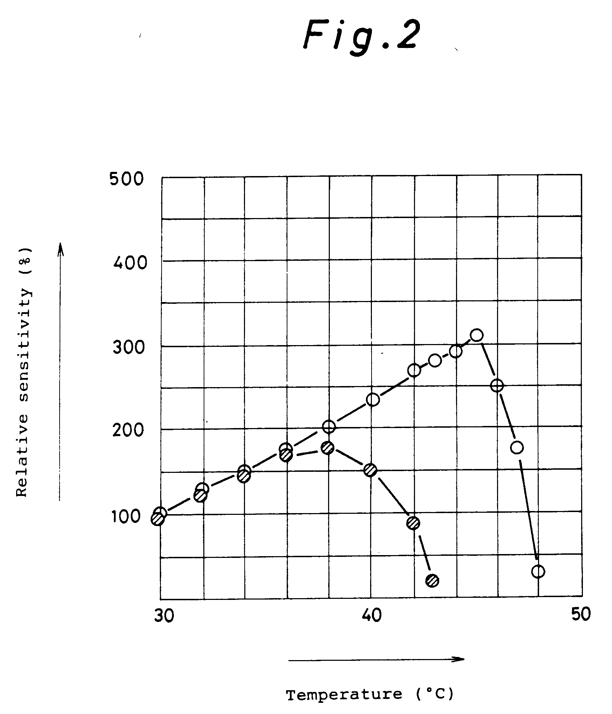

Then the temperature of the thermostat 7 was raised, and the aqueous ethanol solution was added every time a specific temperature had been reached. The respective peak currents are shown in Fig. 2 (○ mark), taking the current at 30 °C as 100 %. In the presence of polyacrylic acid, the high sensitivity exists up to about 45 °C.

### Reference Example 2

### (1) Electrode preparation

Electrodes were prepared in the same manner as in Example 6.

### (2) Measuring method

The measurements were carried out in the same manner as in Example 6, except that no polyacrylic acid was added to the buffer solution.

The results are shown in Fig. 2 ( 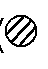 mark). Obviously, the heat resistance was inferior to that of Example 6.

### Example 7

### (1) Electrode preparation

Electrodes were prepared in the same manner as in Example 6.

### (2) Measuring method

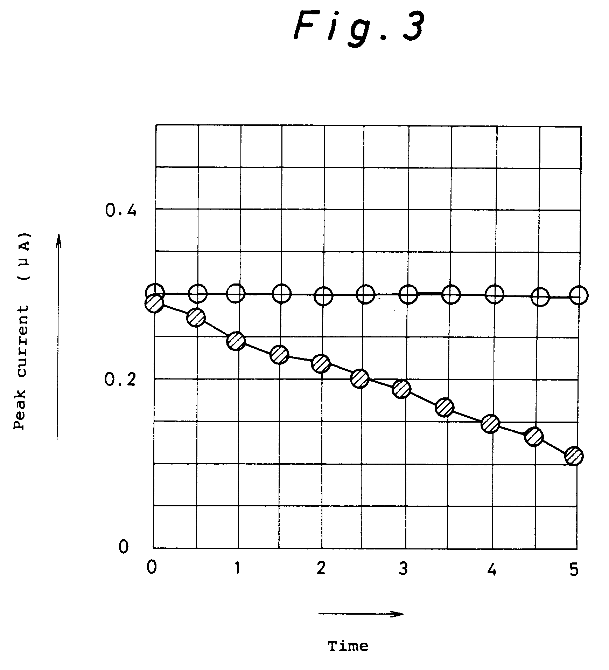

The same measuring apparatus as in Example 6 was used. Setting the thermostat temperature at 40 °C, 5 »l of 50 mM aqueous ethanol solution were injected at specific time intervals, and the peak currents were measured (○ marks in Fig. 3). It may be seen from Fig. 3 that the sensitivity was not changed even after 5 hours.

### Reference Example 3

### (1) Electrode preparation

Electrodes were prepared in the same manner as in Example 6.

### (2) Measuring method

The measurements were carried out in the same manner as in Example 7, except that no polyacrylic acid was added to the buffer solution used in the measurement, by using the same measuring apparatus.

Setting the thermostat temperature at 40 °C, 5 »l of 50 mM aqueous ethanol solution were added at specific time intervals, and the peak currents were measured ( 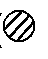 marks in Fig. 3). The sensitivity was not stable and was gradually lowered. The comparison with Example 7 shows the effect of the addition of polyacrylic acid.

### Example 8

### (1) Electrode preparation

The side surface of a platinum wire of 2 mm in diameter was coated with a heat-shrinkable PTFE, and one end of the wire was smoothly finished by using a file and an emery paper of 1500 count. Using this platinum wire as working electrode, a 1 cm² platinum plate as counter electrode and a saturated calomel electrode (SCE) as reference electrode, an electrolysis was conducted for 10 minutes at +1.4 V in 0.1 M sulfuric acid. The platinum wire was washed well in water, dried for 10 minutes at 40 °C, and was dipped for 1 hour into an anhydrous toluene solution containing 10 % of γ-aminopropyl triethoxy silane, and washed. On this amino-silane coated platinum wire, an enzyme was immobilized in the following manner.

To 100 »l of alcohol oxidase (Sigma Co.; derived from Pichia pastolis, liquid enzyme), 5 mg of bovine serum albumin (Sigma Co.; Fraction V) were added, and 1 ml of 100 mM sodium phosphate buffer solution (pH 7.5) saturated with ammonium sulfate was added in ice-chilled state. By this operation, the solution became white and turbid, and the enzyme and albumin became insoluble. This solution was centrifugated for 10 minutes at 4 °C and a relative centrifugal acceleration of 20000. After removal of the supernatant, 100 »l of 100 mM sodium phosphate buffer solution (pH 7.5) were added, and the sedimenting protein was dissolved again. This solution was dialyzed for 1 hour at 4 °C with the same buffer solution as used in the redissolving step. The solution obtained after dialysis was used in the subsequent immobilizing operation. By the operational steps up to dialysis, the sugars and glycerol contained as stabilizers in the enzyme specimen and the ammonium sulfate used for precipitating the protein were removed.

To the solution after dialysis, glutaraldehyde was added in an amount of 0.2 %. 3 »l of this mixed solution were promptly put on the prepared amino-silane coated platinum wire, and dried and cured for 15 minutes at 40 °C. Afterwards, the product was stored in 100 mM sodium phosphate buffer solution (pH 7.5).

### (2) Measuring method

The prepared enzyme electrode used as working electrode was incorporated into a flow-type measuring apparatus shown in Fig. 1. As reference electrode, an Ag|AgCl reference electrode was used, and a stainless steel pipe connected to the flow cell 2 was used as counter electrode 3. These three electrodes were connected to a potentiostat 4. A voltage of +0.6 V against the Ag|AgCl reference electrode was applied to the working electrode 5. The line after the injection port 6 of the flow-type measuring apparatus was put into a thermostat 7. The buffer solution used in the measurement was 100 mM sodium phosphate buffer solution (pH 7.5) containing 50 mM of potassium chloride, 0.01 % of polyacrylic acid and 10 »m of sodium azide.

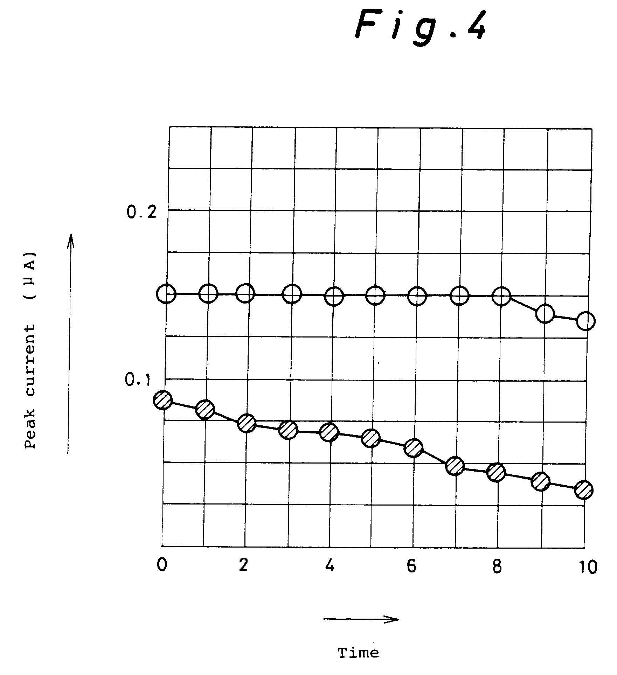

Setting the thermostat temperature at 37 °C, 5 »l of 2 mM aqueous ethanol solution were injected at specific time intervals, and the peak current was measured (○ marks in Fig. 4). It is evident that the sensitivity is not lowered even after 8 hours.

### Reference Example 4

### (1) Electrode preparation

Electrodes were prepared in the same manner as in Example 8.

### (2) Measuring method

The measurements were carried out in the same manner as in Example 8, except that no polyacrylic acid and no sodium azide were added to the phosphate buffer solution used in the measurement, using the same measuring instrument.

Setting the thermostat temperature at 37 °C, 5 »l of 2 mM aqueous ethanol solution were injected at specific time intervals, and the peak current was measured ( 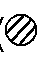 marks in Fig. 4). In this example, the sensitivity was low, not stable, and was gradually lowered. As compared with Example 8, hence, the effects of the addition of polyacrylic acid and sodium azide are evident.

### Example 9

### (1) Electrode preparation

In the same manner as in Example 8, a platinum wire was prepared with immobilized alcohol oxidase.

To the alcohol oxidase solution after dialysis, 5 mM of reduced glutathione and 0.2 % of glutaraldehyde were added. 3 »l of this mixed solution were promptly put on the prepared amino-silane coated platinum wire, and dried and cured at 40 °C for 15 minutes. The electrode was stored in 100 mM sodium phosphate buffer solution (pH 7.5).

### (2) Measuring method

The prepared enzyme electrode used as working electrode was incorporated into a flow type measuring apparatus shown in Fig. 1. As reference electrode, an Ag|AgCl reference electrode 1 was used, and a stainless steel pipe connected to the outlet of the flow cell 2 was used as counter electrode 3. These three electrodes were connected to a potentiostat 4. To the working electrode 5, a voltage of +0.6 V against the Ag|AgCl reference electrode was applied. The line after the injection port 6 of the flow-type measuring apparatus was put into a thermostat 7. The buffer solution used in the measurement was 100 mM sodium phosphate buffer solution (pH 7.5) containing 50 mM of potassium chloride, 0.01 % of polyacrylic acid, and 10 »M of sodium azide.

Setting the thermostat temperature at 37 °C, 5 »l of 2 mM aqueous ethanol solution were injected, and the peak current was recorded. Within the same time range as in Example 8, similarly, there was no change in the sensitivity. The mean and standard deviation values of the sensitivity in 8 hours were 0.321 and ± 0.009 »A, respectively. This result corresponds to about 2.1 times that of Example 8. Thus, by adding reduced glutathione at the time of immobilization, the activity of the immobilized enzyme could be kept high.

## Claims

1. Alcohol oxidase enzyme electrode, comprising an immobilized enzyme layer on a membrane or on a conductive electrode surface, obtainable by applying a solution of an alcohol oxidase, a cross-linking agent and reduced glutathione to the membrane or the conductive electrode surface so as to form the immobilized enzyme layer.

2. The enzyme electrode according to claim 1, wherein the alcohol oxidase is derived from methanol-utilizing microorganisms.

3. The enzyme electrode according to claim 1 or 2, wherein the alcohol oxidase is derived from a methanol-utilizing Candida yeast.

4. The enzyme electrode according to claim 1 or 2, wherein the alcohol oxidase is derived from a methanol-utilizing Pichia yeast.

5. The enzyme electrode according to one of claims 1-4, obtainable with an alcohol oxidase solution, wherein the concentration of reduced glutathione is 0.1 to 10 mM.

6. The enzyme electrode according to one of claims 1-5, obtainable with a multifunctional aldehyde as crosslinking agent.

7. The enzyme electrode according to claim 6, obtainable with glutaraldehyde as multifunctional aldehyde.

8. The enzyme electrode according to claim 6 or 7, obtainable with an alcohol oxidase solution comprising the multifunctional aldehyde in a concentration of 0.1 to 1.0 wt. %.

9. The enzyme electrode according to one of claims 1-8, obtainable with an alcohol oxidase solution further containing at least one type of protein other than alcohol oxidase.

10. The enzyme electrode according to claim 9, obtainable with use of a protein other than alcohol oxidase selected from gelatine, albumin, globulin, polylysine and polyarginine.

11. The enzyme electrode according to claim 9 or 10, obtainable with an amount of the protein of 1/10 to 10 times the weight of the alcohol oxidase.

12. An electrochemical method for the quantitative determination of the alcohol content of a sample in a buffer solution by contacting an enzyme electrode comprising immobilized alcohol oxidase with the solution and measuring the current due to oxygen consumed or due to hydrogen peroxide produced at the time of alcohol oxidation, characterized in that an enzyme electrode according to one of claims 1-11 is used.

13. The method according to claim 12, characterized in that a buffer solution containing sodium azide is used.

14. The method according to claim 13, wherein the concentration of sodium azide in the buffer solution used in the measurement is 0.1 to 1 mM.

15. The method according to one of claims 12-14, characterized in that a buffer solution containing polyacrylic acid is used.

16. The method according to claim 15, wherein the concentration of polyacrylic acid is 0.001 to 0.5 wt.%.

## Patentansprüche

1. Alkoholoxidase-Enzymelektrode, die auf einer Membran oder auf der Oberfläche einer leitfähigen Elektrode eine Schicht mit immobilisiertem Enzym enthält, erhältlich durch Aufbringen einer Lösung einer Alkoholoxidase, eines Vernetzungsmittels und von reduziertem Glutathion auf die Membran oder die Oberfläche der leitfähigen Elektrode, um so die Schicht mit immobilisiertem Enzym zu erzeugen.

2. Enzymelektrode nach Anspruch 1, wobei die Alkoholoxidase aus Mikroorganismen abgeleitet ist, die Methanol verwerten.

3. Enzymelektrode nach Anspruch 1 oder 2, wobei die Alkoholoxidase aus einer Methanol verwertenden Candida-Hefe abgeleitet ist.

4. Enzymelektrode nach Anspruch 1 oder 2, wobei die Alkoholoxidase aus einer Methanol verwertenden Pichia-Hefe abgeleitet ist.

5. Enzymelektrode nach einem der Ansprüche 1 bis 4, erhältlich mit einer Alkoholoxidaselösung, wobei die Konzentration an reduzierten Glutathion 0,1 bis 10 mM beträgt.

6. Enzymelektrode nach einem der Ansprüche 1 bis 5, erhältlich mit einem multifunktionellen Aldehyd als Vernetzungsmittel.

7. Enzymelektrode nach Anspruch 6, erhältlich mit Glutaraldehyd als multifunktionellem Aldehyd.

8. Enzymelektrode nach Anspruch 6 oder 7, erhältlich mit einer Alkoholoxidaselösung, die den multifunktionellen Aldehyd in einer Konzentration von 0,1 bis 1,0 Gew.-% enthält.

9. Enzymelektrode nach einem der Ansprüche 1 bis 8, erhältlich mit einer Alkoholoxidaselösung, die außerdem mindestens einen Protein-Typ außer der Alkoholoxidase enthält.

10. Enzymelektrode nach Anspruch 9, erhältlich unter Verwendung eines Proteins außer der Alkoholoxidase, das unter Gelatine, Albumin, Globulin, Polylysin und Polyarginin ausgewählt ist.

11. Enzymelektrode nach Anspruch 9 oder 10, erhältlich mit einer Proteinmenge, die dem 0,1- bis 10fachen des Gewichts der Alkoholoxidase entspricht.

12. Elektrochemisches Verfahren zur quantitativen Bestimmung des Alkoholgehalts einer Probe in einer Pufferlösung, bei dem eine Enzymelektrode, die immobilisierte Alkoholoxidase enthält, mit der Lösung in Kontakt gebracht wird und bei dem der Strom infolge des Sauerstoffverbrauchs oder der Bildung von Wasserstoffperoxid während der Alkoholoxidation gemessen wird, dadurch gekennzeichnet, daß eine Enzymelektrode nach einem der Ansprüche 1 bis 11 verwendet wird.

13. Verfahren nach Anspruch 12, dadurch gekennzeichnet, daß eine Pufferlösung verwendet wird, die Natriumazid enthält.

14. Verfahren nach Anspruch 13, wobei die Konzentration an Natriumazid in der bei der Messung verwendeten Pufferlösung 0,1 bis 1 mM beträgt.

15. Verfahren nach einem der Ansprüche 12 bis 14, dadurch gekennzeichnet, daß eine Pufferlösung verwendet wird, die Polyacrylsäure enthält.

16. Verfahren nach Anspruch 15, wobei die Konzentration an Polyacrylsäure 0,001 bis 0,5 Gew.-% beträgt.

## Revendications

1. Electrode à enzyme alcool-oxydase, comprenant une couche d'enzyme immobilisée disposée sur une membrane ou sur une surface d'électrode conductrice, que l'on peut obtenir en appliquant une solution d'une alcooloxydase, d'un agent de réticulation et de glutathion réduit à la membrane ou à la surface d'électrode conductrice de façon à former une couche d'enzyme immobilisée.

2. Electrode à enzyme selon la revendication 1, où l'alcool-oxydase est dérivée de micro-organismes utilisant du méthanol.

3. Electrode à enzyme selon la revendication 1 ou 2, où l'alcooloxydase est dérivée d'une levure Candida utilisant du méthanol.

4. Electrode à enzyme selon la revendication 1 ou 2, où l'alcooloxydase est dérivée d'une levure Pichia utilisant du méthanol.

5. Electrode à enzyme selon l'une quelconque des revendications 1 à 4, que l'on peut obtenir avec une solution d'alcool-oxydase, où la concentration en glutathion réduit est de 0,1 à 10 mM.

6. Electrode à enzyme selon l'une quelconque des revendications 1 à 5, que l'on peut obtenir avec un aldéhyde multifonctionnel comme agent de réticulation.

7. Electrode à enzyme selon la revendication 6, que l'on peut obtenir avec du glutaraldéhyde comme aldéhyde multifonctionnel.

8. Electrode à enzyme selon la revendication 6 ou 7, que l'on peut obtenir avec une solution d'alcool-oxydase comprenant l'aldéhyde multifonctionnel à une concentration pondérale de 0,1 à 1,0 %.

9. Electrode à enzyme selon l'une quelconque des revendications 1 à 8, que l'on peut obtenir avec une solution d'alcool-oxydase contenant en outre au moins un type de protéine autre que l'alcool-oxydase.

10. Electrode à enzyme selon la revendication 9, que l'on peut obtenir en utilisant une protéine, autre que l'alcool-oxydase, choisie parmi la gélatine, l'albumine, la globuline, la polylysine et la polyarginine.

11. Electrode à enzyme selon la revendication 9 ou 10, que l'on peut obtenir avec, pour la protéine, une quantité de 1/10 à 10 fois le poids de l'alcooloxydase.

12. Procédé électrochimique destiné à la détermination quantitative de la teneur alcoolique d'un échantillon placé dans une solution tampon par mise en contact, avec la solution, d'une électrode à enzyme comprenant une alcooloxydase immobilisée, et mesure du courant dû à l'oxygène consommé ou dû au peroxyde d'hydrogène produit au moment de l'oxydation alcoolique, caractérisé en ce qu'on utilise une électrode à enzyme selon l'une quelconque des revendications 1 à 11.

13. Procédé selon la revendication 12, caractérisé en ce qu'on utilise une solution tampon contenant de l'azide de sodium.

14. Procédé selon la revendication 13, où la concentration en azide de sodium dans la solution tampon utilisée pour la mesure est de 0,1 à 1 mM.

15. Procédé selon l'une quelconque des revendications 12 à 14, caractérisé en ce qu'on utilise une solution tampon contenant du poly(acide acrylique).

16. Procédé selon la revendication 15, où la concentration pondérale en poly(acide acrylique) est de 0,001 à 0,5 %.
